# EUROPEAN PATENT SPECIFICATION

(11) **EP 3 090 649 B1**
(45) Date of publication and mention of the grant of the patent: **17.07.2019**
(21) Application number: 16159500.4
(22) Date of filing: 09.03.2016
(51) Int. Cl.: A45D 44/18, A61C 17/22, A61C 19/02, A47K 1/09, A61L 2/22, A61L 2/10, A61L 2/26, A61C 19/00

(54) **ELECTRIC ORAL HYGIENE DEVICE STORAGE CONTAINER**
AUFBEWAHRUNGSBEHÄLTER FÜR ELEKTRISCHE MUNDHYGIENEVORRICHTUNG
CONTENANT DE STOCKAGE DE DISPOSITIF D'HYGIENE BUCCO-DENTAIRE

(30) Priority: 28.04.2015 EP 15165390
(43) Date of publication of application: 09.11.2016
(73) Proprietor: Braun GmbH, 61476 Kronberg (DE)
(72) Inventor: Kreßmann, Frank, 61476 Kronberg/Taunus (DE); Müller, Frank, 61476 Kronberg/Taunus (DE); Renz, Anke, 61476 Kronberg/Taunus (DE); Hübner, Ansgar, 61476 Kronberg/Taunus (DE); Reick, Hansjörg, 61476 Kronberg/Taunus (DE); Dietzel, Daniel, 61476 Kronberg/Taunus (DE); Sabisch, Markus, 61476 Kronberg/Taunus (DE); Schubert, Fritz, 61476 Kronberg/Taunus (DE); Kiiver, Egle, 61476 Kronberg/Taunus (DE)
(74) Representative: Schneider, Stefan Michael

(56) References cited:
- JP-A- H0 880 220
- JP-A- H07 263 032
- JP-U- H02 147 040
- US-A- 3 287 076
- US-A- 4 123 845
- US-A1- 2011 100 865
- US-A1- 2014 150 189

## Description

### FIELD OF THE INVENTION

The present invention is concerned with an electric oral hygiene device storage container having a charging unit for charging an electric oral hygiene device disposed in the container and an electrical input connector for providing energy to the charging unit.

### BACKGROUND OF THE INVENTION

It is known that an electric oral hygiene device storage container can be equipped with a charging unit and an electrical input connector for providing energy to the charging unit. Document US 2014/150189 A1 generally discusses such an electric oral hygiene device storage container. This document discloses a hygienic container that stores various personal care items in a clean, hygienic, and inconspicuous manner. The hygienic container includes an inner compartment accessible by means of a hinged door. The inner compartment has multiple drain holes from which fluids can drain as well as various means for storing personal items such as toothbrushes, toothpaste, dental floss, and charging bases. An opening in the back of the inner compartment allows access for electrical cords used in charging various personal items. One or more surfaces or parts thereof comprise magnets such that magnetic items and/or items that include ferrous metals can be attached thereto. The hygienic container also includes feet that lift the bottom surface off the ground, thereby enabling drainage from and air circulation through the inner compartment. A UV germicidal light turns on after the door is closed to disinfect items contained within the hygienic container.

Document US 2011/100865 discloses a storage container for an electrical oral hygiene device comprising the features defined in the preamble of appended claim 1.

It is an object of the present disclosure to provide an electric oral hygiene device storage container that is improved over the known containers or that provides at least an alternate realization of such a container.

### SUMMARY OF THE INVENTION

In accordance with one aspect there is provided an electric oral hygiene device storage container having an removable electric oral hygiene device, a first casing part, at least a second casing part, which first and second casing parts are mechanically coupled to allow for an open container state in which the electric oral hygiene device can be removed from the container and a closed container state in which the electric oral hygiene device is secured inside of the container, wherein the container further has a charging unit for selectively charging the electric oral hygiene device, an electrical input connector realized at an outer side of the first or second casing part for providing energy to the charging unit, and at least one electrical output connector realized at an outer side of the first or second casing part, wherein the charging unit is arranged for selectively providing the energy received at the electrical input connector via the electrical output connector to an external device.

In accordance with one aspect there is provided a kit comprising an electric oral hygiene device storage container as described in the present disclosure and an energy supply unit having an electrical output connector that is connectable with the electrical input connector of the container.

### BRIEF DESCRIPTION OF THE DRAWINGS

The present disclosure will be further elucidated by a detailed description of example embodiments and with reference to figures. In the figures
- Fig. 1: is a depiction of an example electric oral hygiene device storage container in a closed state;
- Fig. 2: is a depiction of the electric oral hygiene device storage container shown in Fig. 1 in its open state, without an electric oral hygiene device stored in the container;
- Fig. 3: is a depiction of an example outer side wall of the electric oral hygiene device storage container shown in Figs. 1 and 2 at which an electrical input connector and an electrical output connector are realized together with further optional elements;
- Fig. 4: is a depiction of the electric oral hygiene device storage container shown in Fig.2 but with an electric oral hygiene device and a replacement head for the electric oral hygiene device accommodated in the electric oral hygiene device storage container;
- Fig. 5: is a depiction of an electric oral hygiene device storage container with indication of various optional elements that may be realized in such a container in example embodiments;
- Fig. 6: is a depiction of a kit comprising an example electric oral hygiene device storage container and an energy supply unit; and
- Fig. 7: is a schematic depiction of various possible electric parts and connections between these parts that may be present in example embodiments of electric oral hygiene device storage containers as proposed.

### DETAILED DESCRIPTION OF THE INVENTION

In accordance with the present disclosure, an electric oral hygiene device storage container (where in the following "electric oral hygiene device storage container" will be abbreviated by "container") comprises, as already mentioned, at least one electrical output connector arranged on an outer side of the first or second part of the casing. This enables to use the container not only for storing the removable electric oral hygiene device and for charging it, but also for charging an external device, e.g. a smartphone, a tablet computer, a pocket lamp, a fitness tracker etc. and thus the versatility of the container is enhanced. A container as described may in particular be used during travel (in addition, the container may be equipped to house further parts such as replacement heads for the electric oral hygiene device, a dental floss reservoir etc.). While the electric oral hygiene device (it is to be understood that the term "electric oral hygiene device" means complete devices but also handles of such devices to which replaceable heads can attached and, where present, at least one replaceable head) is being charged, e.g. in a hotel room after some time of travel, an external device can also easily be connected with the electrical output connector of the container so that carrying along a further charging device for this external device can be dispensed with. The electrical output connector may in particular be arranged as a standardized outlet (e.g. male or female connector or plug), e.g. as an USB or mini USB outlet or as a (e.g. 2.1 mm or 2.5 mm pin size) DC connector. In some embodiments, the electrical output connector is arranged as an inductive electrical output connector (e.g. the electrical output connector may then be designed as an inductive electrical energy transfer unit in accordance with the Qi standard or the electrical output connector may be designed in accordance with the Energy Matters Alliance standard).

The container as described herein comprises a casing having a first casing part and a second casing part that are mechanically connected, e.g. by means of at least one hinge and alternatively or additionally by at least one snap or other type of lock or fastening connection, so that the container can be brought into an open position in which the electric oral hygiene device can be disposed in the container or can be taken out of the container and into a closed position in which the electric oral hygiene device is secured inside of the container, e.g., during travel. In some embodiments, at least one of the first or second casing parts comprises an inlay having a recess for at least accommodating the electric oral hygiene device. The inlay may be arranged to be replaceable, e.g. for cleaning reasons or to adapt the container to differently shaped electric oral hygiene devices (i.e. at least two different inlays are then provided). The inlay may comprise further recesses for receiving other parts such as, e.g., a replacement head for use with the electric oral hygiene device.

The container as described herein comprises an electrical input connector and a charging unit for selectively charging the electric oral hygiene device (the charging unit may be arranged for enabling the charging function only in the closed state of the casing, but in some embodiments charging is also possible in the open state of the casing). While the electrical input connector may be arranged in any known, in particular standardized manner (e.g. as a USB, mini USB or DC connector), the electrical input connector is in some embodiments arranged as a proprietary (i.e. non-standardized) connection. By such a design, the electrical input connector can only be coupled with a respective proprietary (complementary) coupling partner so that only a defined (in particular water-proof) connection can be established between the container and an energy source (e.g. a wall socket). Thus it can be achieved that regulatory and safety requirements are met. The charging unit may provide energy to the electric oral hygiene device in particular in an inductive manner (even though a direct electrical connection is also contemplated). The container may comprise a pin disposed in a complementary recess in the electric oral hygiene device, which pin may serve for positioning the electric oral hygiene device correctly with respect to an inductive energy transmitter (e.g. a coil) of the charging unit.

A kit can be provided comprising a container as described herein and an energy supply unit having an electrical output connector that is connectable with the electrical input connector of the container as has been mentioned before. The electrical safety barrier for usage in a wet environment can then be implemented in the energy supply unit, which transforms the high AC voltage provided by a wall socket into a lower (e.g. 3 to 12 V) DC voltage.

In some embodiments, the charging unit comprises a charging prioritization unit for selectively controlling the supply of energy provided via the electrical input connector to the charging unit and via the charging unit then selectively to the electrical output connector. The charging prioritization unit may be arranged to apply pre-programmed (or hard-wired) rules for selectively providing energy for charging of the electric oral hygiene device and for charging of an external device connected to the electrical output connector (optionally energy may also be provided to further units, e.g. a sanitizing unit, a feedback element, or a powered fan, which units are described in more detail further below). As one rule applied by the charging prioritization unit, energy supplied via the electrical input connector is directly routed to the electrical output connector in case that the electric oral hygiene device has been removed from the container or in case that the electric oral hygiene device is already fully or sufficiently charged (i.e. selection in dependence on the charging state of the electric oral hygiene device). An external device connected with the electrical output connector can then be provided with electrical energy, e.g. for the purpose of charging the external device. A further rule that may be applied by the charging prioritization unit is the following: in case the electric oral hygiene device is still to be charged, energy supply to the electrical output connector may be inhibited or may be controlled to be intermittent so that charging of the electric oral hygiene device is prioritized.

In some embodiments, the container comprises a user input unit (e.g. a switch or a wireless receiver unit for receiving signals from an external device) by which a user can set a prioritization of the charging unit or can override a charging prioritization unit if such is present. The user input unit may even be arranged such that the user can reprogram the charging prioritization unit, e.g. the user input unit may be a Bluetooth receiver that can receive commands from a computer having a Bluetooth transmitter. By such a user input unit it may be achieved that energy is directly provided to the electrical output connector instead of to the charging unit for charging of the electric oral hygiene device to thus manually prioritize the electrical output connector (and thus, e.g., prioritize charging of an external device over charging of the electric oral hygiene device).

In some embodiments, the container comprises an energy source, in particular a rechargeable accumulator or a replaceable energy source such as a battery. The charging unit (which may be controlled by a charging prioritization unit) may use the energy source to charge the electric oral hygiene device and/or an external device via the electrical output connector (in particular in cases when no energy is provided via the electrical input connector). In case of a rechargeable energy source, the charging unit may charge the energy source when it is not fully charged (in particular when the electric oral hygiene device is already fully charged). The charging prioritization unit, if present, may take over the selective control over the charging sequence.

In some embodiments, the container further comprises at least one feedback element for in particular indicating a status of the charging unit (e.g. the status information may comprise at least one of a "no activity" feedback, a "charging of electric oral hygiene device ongoing" feedback, a "charging of electric oral hygiene device completed" feedback, a "energy provided at electrical output connector" feedback", a "charging of internal accumulator ongoing" feedback, or an "internal accumulator is discharged" feedback, where the available type of feedback indications is obviously bound by the kind of configuration of the container - e.g. if no rechargeable accumulator is present, the feedback possibilities do not comprise a feedback that is related to the rechargeable accumulator). The feedback element may be arranged for at least one of an audible, visual, or tactile feedback. The feedback element may be a light emitting element such as a (optionally multi-color) LED, a sound emission element (such as a loudspeaker, but e.g. also a motor may be used to generate a sound), or a vibrator element (e.g. a piezo-vibrator). The feedback element may be arranged on an outer side (outer surface) of the container, in particular if it is a visual feedback element. In some embodiments, the feedback element is arranged at the removable electric oral hygiene device, which is used to take over the feedback function, e.g. an LED provided at the housing (which may be visible through the closed container) of the electric oral hygiene device may be used for visual feedback or a motor of the electric oral hygiene device may be used for tactile and/or audible feedback.

In some embodiments, the container comprises a sanitizing unit arranged for sanitizing at least a part of the electric oral hygiene device, e.g. a functional head of the electric oral hygiene device such as a brush head (which functional head may be attachable to and detachable from a handle of the electric oral hygiene device and which functional head may in particular be received in the container being detached from the handle). For example, the sanitizing unit may comprise an ejector unit (e.g. a piezo-element driven ejector nozzle) for ejecting a small amount of a sanitizing fluid (e.g. in the form of a fine spray) onto at least the part of the electric oral hygiene device to be sanitized when the container is closed. Alternatively or additionally, the sanitizing unit may comprise a UV light source for emission of germicidal ultraviolet radiation towards at least the part of the electric oral hygiene device to be sanitized.

In some embodiments, the container is arranged so that a display device can be supported by the container when the container is in the open position. The container is then equipped with at least one receiving structure for holding the display device (in particular in an upright or slightly inclined position). The at least one holding structure may in particular be arranged to hold in place a display device of a particular geometry. In some embodiments, at least two receiving are present, which may comprise a cut-out provided in the first casing part and a recess in the second casing part, where the recess may in particular be provided in a replaceable inlay so that the recess can be adapted to the size of the display device to be received. The display device to be held may in particular be a smartphone.

In some embodiments, the container comprises a humidity removal system. The humidity removal system may comprise active and/or passive means of humidity removal. In some embodiments, a reservoir of moisture absorbing material is provided as humidity removal system. The reservoir may then be arranged to be replaceable (e.g. the reservoir may be snap-connected to the container) or the reservoir may be arranged so that the moisture absorbing material can be replaced. The moisture absorbing material may be a silica gel, but other known moisture absorbing materials are possible as well. Additionally or alternatively, the container comprises an active and/or passive venting system as humidity removal system, e.g. at least one venting opening and/or a powered fan.

In some embodiments, the container is equipped with at least one replaceable inlay. E.g. at least one of the casing parts may be arranged to have a generally shell-like structure in which an inlay may be snap-fitted or press-fitted. Such an inlay may be adapted to a particular electric oral hygiene device. The inlay may comprise deformable foam material to allow adaptation to differently shaped electric oral hygiene devices.

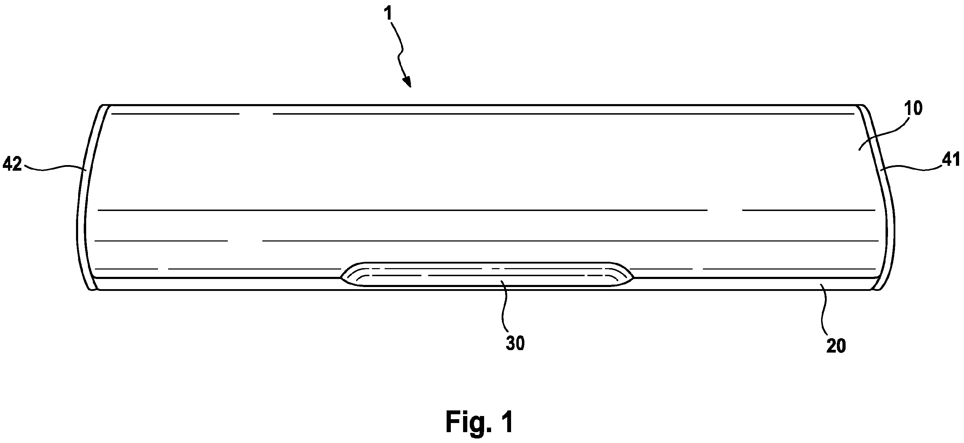

Fig. 1 is a depiction of an example container 1 as proposed in a closed state. The container 1 has a first casing part 10 and a second casing part 20. In the shown embodiment, an optional locking element 30 keeps the first and second casing parts 10, 20 together in the closed state and a user may need to press against the locking element 30 to release the locking function and to open the container 1. A spring element may be arranged to push the first and second casing parts 10, 20 apart once the locking function is released. The locking element 30 may be arranged at either one of the first and second casing parts 10, 20. In the shown embodiment, the container 1 has side walls 41, 42 that may be attached to or integrally connected with either one of the first or second casing parts 10, 20. Generally, a locking function may be achieved by any suitable means, e.g. by a snap hook, a magnetic lock, a hook and loop fastener, an adhesive connection, a press-fit connection etc.

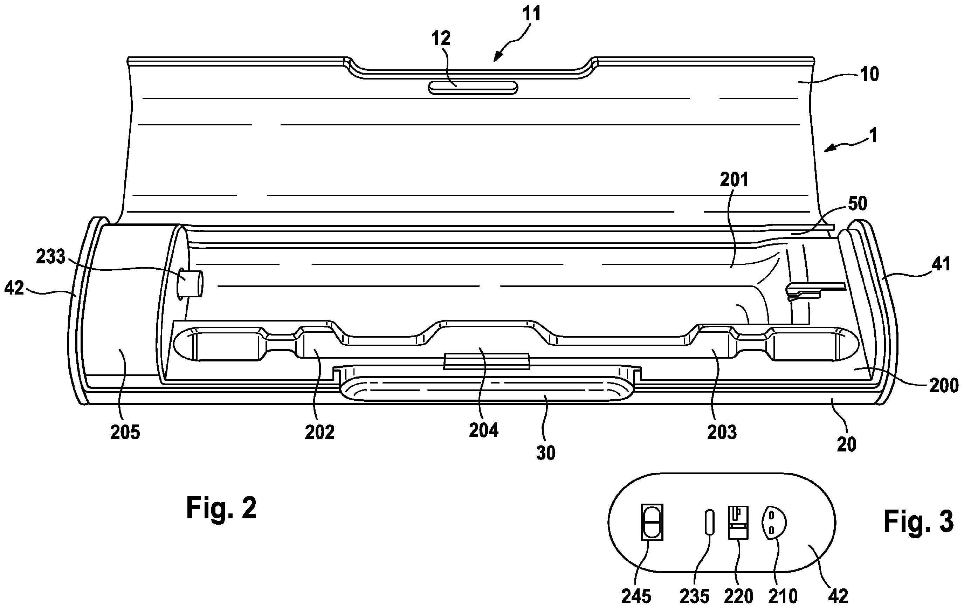

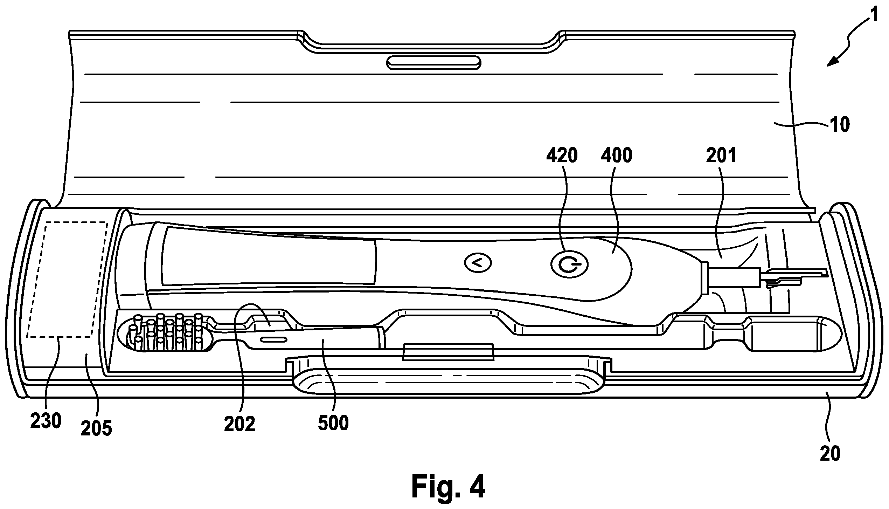

Fig. 2 is a depiction of a container 1 as shown in Fig. 1 but in an open state in which the first casing part 10 (taking the function of a lid or cover) is in a raised position. The removable electric oral hygiene device is not shown in Fig. 2 for sake of visibility other parts of the container 1. Fig. 4 is a picture of the complete container with the removable electric oral hygiene disposed in the inner cavity of the container. The first casing part 10 may have a cut-out 11 that may serve to receive a display device (e.g. a smartphone) to keep the display device in an upright or inclined position. In the shown embodiment, a through-hole 12 is provided in the first casing part 10, which through-hole 12 may serve to receive a projection of the locking element 30. A strip 50 of thin material serves in the shown embodiment as hinge (here: a film hinge) arranged between the first casing part 10 and the second casing part 20. Any other type of hinge is also contemplated, e.g. a living hinge, a pivot hinge, a friction hinge, a self-closing hinge etc.

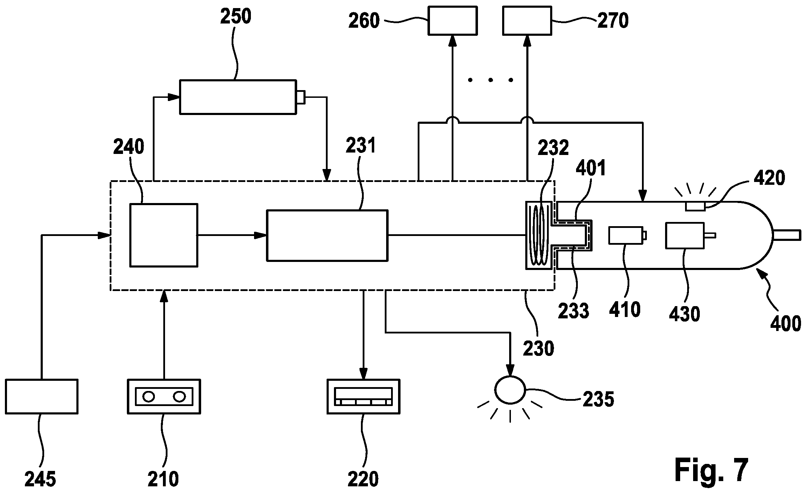

The second casing part 20 serves in the shown embodiment as storage portion of the container 1. The second casing part 20 together with the side walls 41 and 42 forms a shell-like tray. An inlay 200 is inserted into this tray. The inlay 200 is in particular a replaceable inlay so that it can be cleaned or replaced by another inlay to adapt the container to receive other items or to replace a soiled inlay by a fresh inlay. The inlay 200 has four depressions 201, 202, 203, 204 of which depressions 202, 203, and 204 are interconnected. Generally, the inlay 200 may have at least one depression, but any other number of depressions is possible as well. A depression 201 is shaped for receiving the removable electric oral hygiene device (see Fig. 4). The three interconnected depressions 202, 203, and 204 are shaped to receive replacement heads for the electric oral hygiene device (see Fig. 4) in the outer depressions 202 and 203. The center portion 204 of the interconnected depression is shaped so that it can receive a display device, which would then be supported by the center portion 204 of the interconnected depression and optionally also by the cutout 11 in the first casing part. On the left hand side of the depicted container example, a box 205 fills the container, which box 205 in the shown embodiment is arranged to house a charging unit and optionally at least one further electric part as will be explained in the following. In some embodiments, a charging unit is housed in free space under the inlay 200 or in the first casing part 10 etc. A pin 233 extends from the box 205, which pin 233 is shaped so that it can be received in a recess of the electric oral hygiene device to be received in the depression 201. The pin 233 then serves to hold the electric oral hygiene device in an optimal position for inductive charging. While reference is made to a charging unit and optionally at least one further optional electric part that may be housed in box 205 in the following description referring to Figs. 3 to 6, some example embodiments of the charging unit and the at least one optional further electric part of the container 1 are described in connection with Fig. 7 further below.

Fig. 3 is a depiction of the outer side of side wall 42 arranged on the left hand side of the container (i.e. the side at which the box 205 housing the charging unit is arranged) shown in Fig. 2. The side wall 42 is part of the outer side of the container and is a portion of the second casing part 20. The side wall 42 has an electrical input connector 210, an electrical output connector 220, an optional feedback element 235 that may be realized as a light emission element, but which could alternatively or additionally be realized as an audible feedback element (e.g. a loudspeaker) or a tactile feedback element (e.g. a vibrator element), and an optional user input unit 245 here realized as a switch for manually overriding a standard charging scheme as is explained in connection with Fig. 7 below. Generally, an electrical input connector and an electrical output connector (as well as the further optional elements) may each be arranged at any outer side of the container and Fig. 3 is just showing a particular example.

Fig. 4 is a depiction of the container shown in Fig. 2 with an electric oral hygiene device 400 (here: a handle of an electric toothbrush) received in depression 201. The electric oral hygiene device 400 may have an illuminated switch 420. By a dashed line it is indicated that box 205 can comprise a charging unit 230 that receives energy at least via an electrical input connector 210 as, e.g., shown in Fig. 3 for in particular charging a rechargeable battery provided in the electric oral hygiene device 400 and which charging unit 230 is arranged to also selectively provide energy at the electrical output connector 220 (as, e.g., shown in Fig. 3). A replacement head 500 for the electric oral hygiene device 400 is received in depression 202.

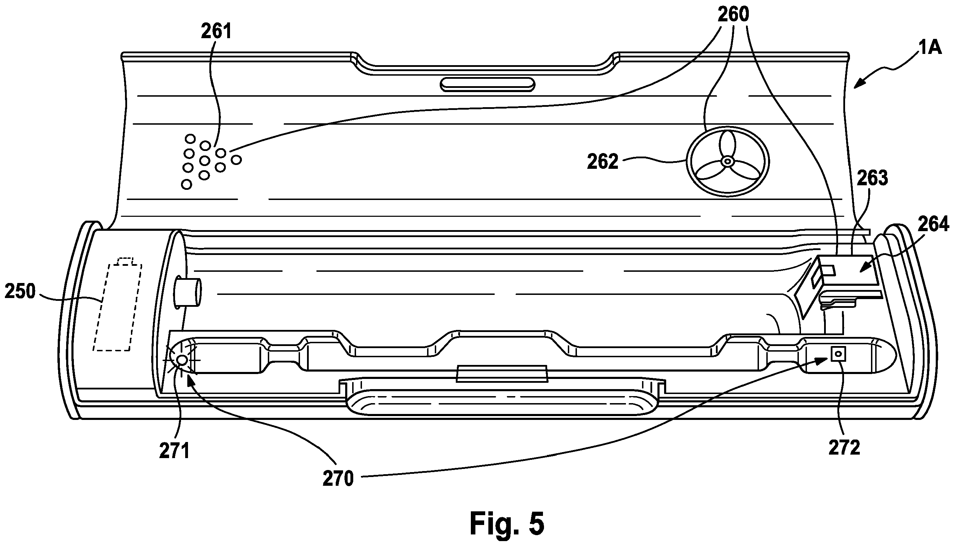

Fig. 5 is a depiction of an example container 1A having several optional features. It is indicated that the container 1A may comprise an accumulator (rechargeable battery) - drawn in dashed lines - for storing energy so that powered elements of the container 1A can be provided with energy even in circumstances where a connection to an external energy source (e.g. wall socket) is not present. It is shown by way of example that the container 1A may comprise a humidity removal system 260 and/or a sanitizing unit 270. The humidity removal system 260 may comprise at least one of the following: (1) at least one venting opening 261, (2) an actively driven venting unit 262 - here realized as a fan, or (3) a reservoir 263 comprising humidity absorbing material 264. The sanitizing unit 270 may comprise at least one of the following: (1) a UV light emitting element 271 (e.g. an UV LED) or (2) a spray unit for generating a spray of sanitizing liquid (e.g. a piezo driven spray nozzle), which spray unit may comprise a reservoir of sanitizing liquid.

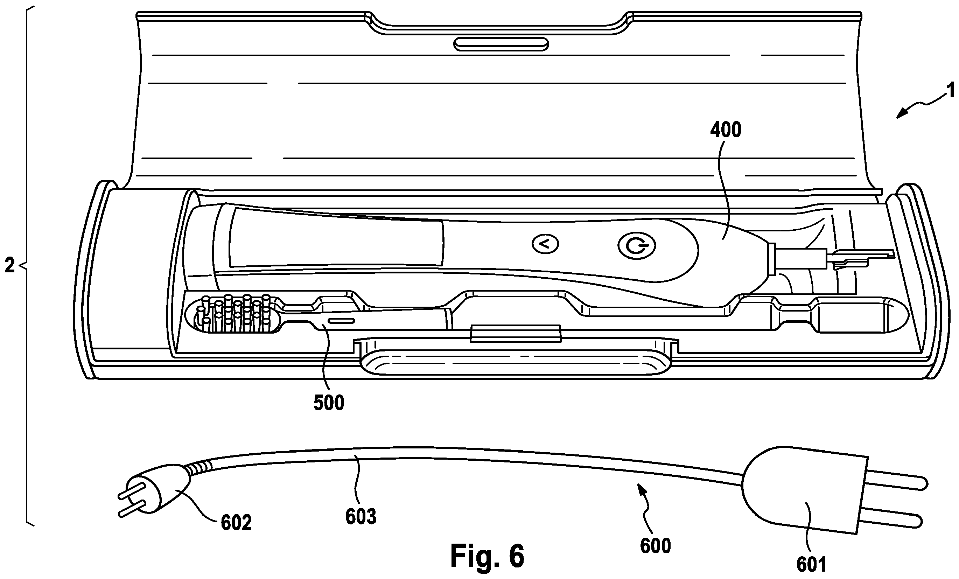

Fig. 6 is a depiction of a kit 2 comprising a container 1 as proposed housing an electric oral hygiene device 400 (realized again as a handle) and a replacement head 500 for the electric oral hygiene device 400 and an energy supply unit 600, which here has a energy plug 601 in which an AC-DC or an AC-AC transformer is included so that e.g. a 12 Volt DC or AC voltage can be provided at the electrical input connector 210 (Fig. 3) of the container 1 via an electrical output connector 602 of the energy supply unit 600. In particular, the electrical output connector 602 of the energy supply unit 600 and the electrical input connector 210 of the container 1 are realized as complementary non-standard connectors to assure that only an energy supply unit 600 providing a water-proof connection can be connected to the container 1, which container 1 may typically be placed in the bathroom.

Fig. 7 is a schematic depiction of the possible electrical parts and electrical connections of a container as discussed herein. This depiction shows various possibilities that do not necessarily need to be present in all embodiments of a proposed container and arbitrary combinations of the optional features are to be considered. A proposed container comprises an electrical input connector 210, an electrical output connector 220 arranged on an outer side of the casing and a charging unit 230 for selectively charging an electric oral hygiene device 400 that comprises a rechargeable energy source 410 such as a rechargeable battery (accumulator or secondary battery). The electrical input connector 210 is arranged for receiving energy from an energy source such as from a wall socket via, e.g., an AC-DC energy converter realizing an energy supply unit. The electrical input connector 210 is coupled with the charging unit 230 to provide the energy to the charging unit 230. The charging unit 230 is coupled with the electrical output connector 220 and is arranged to selectively provide energy at the electrical output connector 220.

In some embodiments the container comprises at least one further electric part connected with at least one of the previously mentioned parts such as a charging prioritization unit 240, a user input unit 245, a feedback element 235, a rechargeable battery 250, a humidity removal system 260 and/or a sanitizing unit 270 that may each be electrically coupled with the charging unit 230. A container as proposed may comprise one or several of these further electric parts and respective electric connections.

In some embodiments, the charging unit 230 comprises a charging circuit 231 and a charging coil 232 for wireless, inductive energy transfer to a charging coil of the electric oral hygiene device 400, where the charging coil 232 may be arranged in a pin-like structure 233 adapted for sliding into a form-fitting recess 401 of the electric oral hygiene device 400 so that the electric oral hygiene device 400 has a defined position to the charging coil 232 during charging.

In some embodiments, the charging unit 230 comprises a charging prioritization unit 240 for prioritizing the provision of energy received via the electrical input connector 210 or alternatively or additionally drawn from a rechargeable energy source 250. The prioritization unit 240 may apply at least one of the following prioritization rules, where these rules may be present as original default settings or may be chosen via the user input unit (which rules are given as examples - any other prioritization rule may be applied as well):
1. First charge the electric oral hygiene device 400 until it is fully charged (or until it is charged to a predetermined degree, e.g. 80%) before energy is provided via the energy outlet 220 for charging an external device. The provision of energy via the electrical output connected is thus controlled in dependence on the charging state of the electrical oral hygiene device.
2. Intermittently provide energy for charging of the electric oral hygiene device 400 until it is fully charged (or until it is charged to a predetermined degree, e.g. 80%) with a first predetermined interval ratio, e.g. 50% or 80% and provide energy via the energy outlet 220 at a second predetermined interval ration of e.g. 50% (if the first predetermined interval ratio is 50%) or less than 50% (if the first predetermined interval ratio is 50%) and in this case use the remaining e.g. 10% for e.g. charging a rechargeable battery 250 (if such is present) or for energizing other loads of the container (e.g. a powered fan of a humidity removal system 260 or a UV light source of a sanitizing unit 270, if one of such is present).
3. First provide energy received via the energy inlet 210 at the energy outlet 220 if a load is present at the energy outlet 220.
4. Draw energy from internal rechargeable battery 250 if no energy is provided via electrical input connector 210.

A user input unit 245 may be provided for switching between prioritization rules, for overriding a prioritization rule by a user defined prioritization rule or for reprogramming the prioritization unit 240. The user input unit 245 may thus be realized as a simple switch to switch between predefined prioritization rules or, e.g., as a Bluetooth based receiving device for wireless reprogramming of the prioritization unit 240 via an external device such as a smartphone (to which a respective reprogramming app has been loaded), tablet, or a computer.

The container may have a feedback element 235 for indicating, e.g., a charging state as has already been described. The feedback element 235 may be arranged at the outer side of the container. In some embodiments, the charging unit 230 is controllably coupled with the electric oral hygiene device 400 so that the charging unit 230 can use a feedback element arranged at the removable electric oral hygiene device 400, e.g. a light emission element 420 or a motor 430 of the electric oral hygiene device 400 to provide visual, acoustic or tactile feedback.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An electric oral hygiene device storage container comprising:
a removable electric oral hygiene device;
a first casing part (10);
at least a second casing part (20);
which first and second casing parts are mechanically coupled to allow for an open container state in which the electric oral hygiene device can be removed from the container and a closed container state in which the electric oral hygiene device is secured inside of the container;
wherein the container further has a charging unit (230) for selectively charging the electric oral hygiene device, an electrical input connector (210) realized at an outer side of the first or second casing part (10, 20) for providing energy to the charging unit, and at least one electrical output connector, wherein the charging unit is arranged for selectively providing the energy received at the electrical input connector via the electrical output connector to an external device,
**characterised in that** the at least one electrical output connector (220) is realized at an outer side of the first or second casing part (10, 20).

2. The electric oral hygiene device storage container in accordance with claim 1, wherein the electrical output connector is designed as a standardized connector such as an USB connector.

3. The electric oral hygiene device storage container in accordance with any one of claims 1 to 2, wherein the charging unit comprises a charging prioritization unit (240) arranged to control the supply of energy provided via the electrical input connector to the charging unit and to the electrical output connector, in particular wherein the charging prioritization unit is arranged to control the supply of energy in dependence on the charging state of the electric oral hygiene device.

4. The electric oral hygiene device storage container in accordance with the previous claim, further comprising a user input unit (245) arranged for manually overriding the charging prioritization unit.

5. The electric oral hygiene device storage container in accordance with any one of claims 1 to 4, further comprising at least one feedback element (235) for providing at least one of an audible, visual, or tactile feedback about the status of the charging unit and/or a charging process performed by the charging unit, in particular where the feedback element is arranged on an outer side of the container.

6. The electric oral hygiene device storage container in accordance with the previous claim, wherein the charging unit or the charging prioritization unit is arranged to use as the feedback element a feedback element arranged at the electric oral hygiene device to provide the feedback.

7. The electric oral hygiene device storage container in accordance with any one of claims 1 to 6, further comprising an internal energy source, in particular a rechargeable accumulator (250) or a replaceable battery.

8. The electric oral hygiene device storage container in accordance with the previous claim, wherein the charging unit is arranged to selectively charge the accumulator when energy is provided via the electrical input connector or to provide energy via the electrical output connector drawn from the accumulator.

9. The electric oral hygiene device storage container in accordance with any one of claims 1 to 8, further comprising a sanitizing unit (270) for sanitizing at least a portion of the electric oral hygiene device.

10. The electric oral hygiene device storage container in accordance with the previous claim, wherein the sanitizing unit (270) comprises a UV light source.

11. The electric oral hygiene device storage container in accordance with any one of claims 1 to 10, wherein the container has at least one receiving structure for holding a removable display device such as a smart phone, in particular wherein at each of the first and second casing parts a receiving structure is arranged to hold the removable display device in the open position of the container.

12. The electric oral hygiene device storage container in accordance with the previous claim, wherein the receiving structures comprise a cut-out provided in the first casing part and a recess in the second casing part, in particular wherein the second casing part comprises an inlay in which the recess is provided.

13. The electric oral hygiene device storage container in accordance with any one of claims 1 to 12, further comprising a humidity removal system (260) and/or an active or passive venting system such as at least one venting slit (261) or a powered fan (262), in particular wherein the humidity removal system comprises a reservoir of moisture absorbing material, in particular wherein the reservoir is arranged to be replaceable or is arranged such that the moisture absorbing material can be replaced.

14. The electric oral hygiene device storage container in accordance with any one of claims 1 to 13, further comprising at least one replaceable inlay (200) for receiving at least the electric oral hygiene device.

15. A kit comprising an electric oral hygiene device storage container in accordance with any one of claims 1 to 14 and an energy supply unit having an electrical output connector that is connectable with the electrical input connector of the container.

## Patentansprüche

1. Aufbewahrungsbehälter einer elektrischen Mundhygienevorrichtung, umfassend:
eine entfernbare elektrische Mundhygienevorrichtung;
einen ersten Gehäuseteil (10);
wenigstens einen zweiten Gehäuseteil (20);
wobei der erste und zweite Gehäuseteil mechanisch gekoppelt sind, um einen offenen Behälterzustand, in dem die elektrische Mundhygienevorrichtung aus dem Behälter entfernt werden kann, und einen geschlossenen Behälterzustand zu ermöglichen, in welchem die elektrische Mundhygienevorrichtung innerhalb des Behälters befestigt ist;
wobei der Behälter ferner eine Ladeeinheit (230) zum selektiven Aufladen der elektrischen Mundhygienevorrichtung, einen elektrischen Eingangsanschluss (210), der an einer äußeren Seite des ersten oder zweiten Gehäuseteils (10, 20) gebildet ist, um Energie an die Ladeeinheit bereitzustellen, und wenigstens einen elektrischen Ausgangsanschluss aufweist, wobei die Ladeeinheit vorgesehen ist, um die Energie, die am elektrischen Eingangsanschluss aufgenommen wird, über den elektrischen Ausgangsanschluss an eine externe Vorrichtung bereitzustellen,
**dadurch gekennzeichnet, dass** der wenigstens eine elektrische Ausgangsanschluss (220) an einer äußeren Seite des ersten oder zweiten Gehäuseteils (10, 20) gebildet ist.

2. Aufbewahrungsbehälter einer elektrischen Mundhygienevorrichtung nach Anspruch 1, wobei der elektrische Ausgangsanschluss als ein standardisierter Anschluss, wie ein USB-Anschluss, ausgebildet ist.

3. Aufbewahrungsbehälter einer elektrischen Mundhygienevorrichtung nach einem der Ansprüche 1 bis 2, wobei die Ladeeinheit eine Ladepriorisierungseinheit (240) umfasst, die dazu vorgesehen ist, die Zufuhr von Energie über den elektrischen Eingangsanschluss an das Ladegerät und an den elektrischen Ausgangsanschluss zu steuern, wobei die Ladepriorisierungseinheit insbesondere dazu vorgesehen ist, die Zufuhr von Energie in Abhängigkeit vom Ladezustand der elektrischen Mundhygienevorrichtung zu steuern.

4. Aufbewahrungsbehälter einer elektrischen Mundhygienevorrichtung nach dem vorstehenden Anspruch, ferner umfassend eine Benutzereingabeeinheit (245), die zum manuellen Außer-Kraft-Setzen der Ladepriorisierungseinheit vorgesehen ist.

5. Aufbewahrungsbehälter einer elektrischen Mundhygienevorrichtung nach einem der Ansprüche 1 bis 4, ferner umfassend wenigstens ein Rückkopplungselement (235), um wenigstens eine von einer akustischen, visuellen oder taktilen Rückmeldung über den Status der Ladeeinheit und/oder eines Ladevorgangs bereitzustellen, der von der Ladeeinheit durchgeführt wird, insbesondere wenn das Rückkopplungselement an einer äußeren Seite des Behälters angeordnet ist.

6. Aufbewahrungsbehälter einer elektrischen Mundhygienevorrichtung nach dem vorstehenden Anspruch, wobei die Ladeeinheit oder die Ladepriorisierungseinheit dazu vorgesehen ist, als das Rückkopplungselement ein Rückkopplungselement zu verwenden, das an der elektrischen Mundhygienevorrichtung vorgesehen ist, um die Rückkopplung bereitzustellen.

7. Aufbewahrungsbehälter einer elektrischen Mundhygienevorrichtung nach einem der Ansprüche 1 bis 6, ferner umfassend eine interne Energiequelle, insbesondere einen wiederaufladbaren Akkumulator (250) oder eine austauschbare Batterie.

8. Aufbewahrungsbehälter einer elektrischen Mundhygienevorrichtung nach dem vorstehenden Anspruch, wobei die Ladeeinheit dazu vorgesehen ist, selektiv den Akkumulator aufzuladen, wenn Energie über den elektrischen Eingangsanschluss bereitgestellt wird, oder aus dem Akkumulator bezogene Energie über den elektrischen Ausgangsanschluss bereitzustellen.

9. Aufbewahrungsbehälter einer elektrischen Mundhygienevorrichtung nach einem der Ansprüche 1 bis 8, ferner umfassend eine Desinfektioneinheit (270) zur Desinfektion von wenigstens einem Teil der elektrischen Mundhygienevorrichtung.

10. Aufbewahrungsbehälter einer elektrischen Mundhygienevorrichtung nach dem vorstehenden Anspruch, wobei die Desinfektionseinheit (270) eine UV-Lichtquelle umfasst.

11. Aufbewahrungsbehälter einer elektrischen Mundhygienevorrichtung nach einem der Ansprüche 1 bis 10, wobei der Behälter wenigstens eine Aufnahmestruktur zum Halten einer entfernbaren Anzeigevorrichtung, wie eines Smartphones, aufweist, wobei insbesondere an jedem des ersten und zweiten Gehäuseteils eine Aufnahmestruktur angeordnet ist, um die entfernbare Anzeigevorrichtung in der offenen Position des Behälters zu halten.

12. Aufbewahrungsbehälter einer elektrischen Mundhygienevorrichtung nach dem vorstehenden Anspruch, wobei die Aufnahmestrukturen eine Aussparung, die im ersten Gehäuseteil bereitgestellt ist, und eine Vertiefung in dem zweiten Gehäuseteil umfassen, wobei insbesondere der zweite Gehäuseteil eine Einlage umfasst, in der die Vertiefung bereitgestellt ist.

13. Aufbewahrungsbehälter einer elektrischen Mundhygienevorrichtung nach einem der Ansprüche 1 bis 12, ferner umfassend ein Feuchtigkeitsentfernungssystem (260) und/oder eine aktives oder passive Belüftungseinrichtung wie wenigstens einen Entlüftungsschlitz (261) oder einen elektrisch angetriebenen Ventilator (262), wobei insbesondere das Feuchtigkeitsentfernungssystem ein Reservoir aus Feuchtigkeit absorbierendem Material umfasst, wobei das Reservoir insbesondere auswechselbar vorgesehen ist oder derart vorgesehen ist, dass das Feuchtigkeit absorbierende Material ersetzt werden kann.

14. Aufbewahrungsbehälter einer elektrischen Mundhygienevorrichtung nach einem der Ansprüche 1 bis 13, ferner umfassend wenigstens eine austauschbare Einlage (200) zur Aufnahme von wenigstens einer elektrischen Mundhygienevorrichtung.

15. Kit, umfassend einen Aufbewahrungsbehälter einer elektrischen Mundhygienevorrichtung nach einem der Ansprüche 1 bis 14 und eine Energiezufuhreinheit mit einem elektrischen Ausgangsanschluss, der mit dem elektrischen Eingangsanschluss des Behälters verbindbar ist.

## Revendications

1. Récipient de stockage pour dispositif d'hygiène buccale électrique comprenant :
un dispositif d'hygiène buccale électrique amovible ;
une première partie (10) de boîtier ;
au moins une deuxième partie (20) de boîtier ;
dont les première et deuxième parties de boîtier sont couplées de façon mécanique pour permettre un état de récipient ouvert dans lequel le dispositif d'hygiène buccale électrique peut être retiré du récipient et un état de récipient fermé dans lequel le dispositif d'hygiène buccale électrique est maintenu à l'intérieur du récipient ;
dans lequel le récipient a en outre une unité de charge (230) pour charger sélectivement le dispositif d'hygiène buccale électrique, un connecteur d'entrée électrique (210) réalisé au niveau d'un côté externe de la première ou de la deuxième partie de boîtier (10, 20) pour fournir de l'énergie à l'unité de charge, et au moins un connecteur de sortie électrique, dans lequel l'unité de charge est agencée pour fournir sélectivement l'énergie reçue au niveau du connecteur d'entrée électrique par le biais du connecteur de sortie électrique à un dispositif externe,
**caractérisé en ce que** le au moins un connecteur de sortie électrique (220) est réalisé au niveau d'un côté externe de la première ou de la deuxième partie de boîtier (10, 20).

2. Récipient de stockage pour dispositif d'hygiène buccale électrique selon la revendication 1, dans lequel le connecteur de sortie électrique est conçu comme un connecteur standardisé tel qu'un connecteur USB.

3. Récipient de stockage pour dispositif d'hygiène buccale électrique selon l'une quelconque des revendications 1 à 2, dans lequel l'unité de charge comprend une unité de priorisation de charge (240) agencée pour contrôler l'alimentation d'énergie fournie par le biais du connecteur d'entrée électrique à l'unité de charge et au connecteur de sortie électrique, en particulier dans lequel l'unité de priorisation de charge est agencée pour contrôler l'alimentation d'énergie en fonction de l'état de charge du dispositif d'hygiène buccale électrique.

4. Récipient de stockage pour dispositif d'hygiène buccale électrique selon la revendication précédente, comprenant en outre une unité d'entrée utilisateur (245) agencée pour ignorer manuellement l'unité de priorisation de charge.

5. Récipient de stockage pour dispositif d'hygiène buccale électrique selon l'une quelconque des revendications 1 à 4, comprenant en outre au moins un élément de retour d'information (235) pour fournir au moins l'un d'un retour d'information audible, visuel ou tactile sur l'état de l'unité de charge et/ou d'un processus de charge effectué par l'unité de charge, en particulier où l'élément de retour d'information est agencé au niveau d'un côté externe du récipient.

6. Récipient de stockage pour dispositif d'hygiène buccale électrique selon la revendication précédente, dans lequel l'unité de charge ou l'unité de priorisation de charge est agencée pour utiliser comme élément de retour d'information un élément de retour d'information agencé au niveau du dispositif d'hygiène buccale électrique pour fournir le retour d'information.

7. Récipient de stockage pour dispositif d'hygiène buccale électrique selon l'une quelconque des revendications 1 à 6, comprenant en outre une source d'énergie interne, en particulier un accumulateur rechargeable (250) ou une batterie remplaçable.

8. Récipient de stockage pour dispositif d'hygiène buccale électrique selon la revendication précédente, dans lequel l'unité de charge est agencée pour charger sélectivement l'accumulateur lorsque de l'énergie est fournie par le biais du connecteur d'entrée électrique ou pour fournir de l'énergie par le biais du connecteur de sortie électrique tirée de l'accumulateur.

9. Récipient de stockage pour dispositif d'hygiène buccale électrique selon l'une quelconque des revendications 1 à 8, comprenant en outre une unité de désinfection (270) pour désinfecter au moins une partie du dispositif d'hygiène buccale électrique.

10. Récipient de stockage pour dispositif d'hygiène buccale électrique selon la revendication précédente, dans lequel l'unité de désinfection (270) comprend une source de lumière UV.

11. Récipient de stockage pour dispositif d'hygiène buccale électrique selon l'une quelconque des revendications 1 à 10, dans lequel le récipient a au moins une structure de réception pour maintenir un dispositif d'affichage amovible tel qu'un smartphone, en particulier dans lequel au niveau de chacune des première et deuxième parties de boîtier une structure de réception est agencée pour maintenir le dispositif d'affichage amovible dans la position ouverte du récipient.

12. Récipient de stockage pour dispositif d'hygiène buccale électrique selon la revendication précédente, dans lequel les structures de réception comprennent une découpe constituée dans la première partie de boîtier et une cavité dans la deuxième partie de boîtier, en particulier dans lequel la deuxième partie de boîtier comprend un encastrement dans lequel la cavité est constituée.

13. Récipient de stockage pour dispositif d'hygiène buccale électrique selon l'une quelconque des revendications 1 à 12, comprenant en outre un système d'élimination d'humidité (260) et/ou un système de ventilation actif ou passif tel qu'au moins l'un d'une fente de ventilation (261) ou d'un ventilateur alimenté (262), en particulier dans lequel le système d'élimination d'humidité comprend un réservoir de matériau absorbant l'humidité, en particulier dans lequel le réservoir est agencé pour être ou remplaçable ou est agencé de telle sorte que le matériau absorbant l'humidité puisse être remplacé.

14. Récipient de stockage pour dispositif d'hygiène buccale électrique selon l'une quelconque des revendications 1 à 13, comprenant en outre au moins une incrustation remplaçable (200) destinée à recevoir au moins le dispositif d'hygiène buccale électrique.

15. Kit comprenant un récipient de stockage pour dispositif d'hygiène buccale électrique selon l'une quelconque des revendications 1 à 14 et une unité d'alimentation d'énergie ayant un connecteur de sortie électrique qui peut être connecté avec le connecteur d'entrée électrique du récipient.
